# EUROPÄISCHE PATENTSCHRIFT

(11) **EP 3 265 013 B1**
(45) Veröffentlichungstag und Bekanntmachung des Hinweises auf die Patenterteilung: **04.12.2019**
(21) Anmeldenummer: 16713739.7
(22) Anmeldetag: 04.03.2016
(51) Int. Cl.: A61C 1/00, A61B 18/20, A61C 19/06, A61B 18/00

(54) **DENTALLASER ZUR BEHANDLUNG VON WEICHGEWEBE**
DENTAL LASER FOR THE TREATMENT OF SOFT TISSUE
LASER DENTAIRE POUR LE TRAITEMENT DES TISSUS MOUS

(30) Priorität: 04.03.2015 DE 102015203881
(43) Veröffentlichungstag der Anmeldung: 10.01.2018
(73) Patentinhaber: Sirona Dental Systems GmbH, 64625 Bensheim (DE)
(72) Erfinder: SUTTER, Ralf, 69469 Weinheim (DE); OEHME, Bernd, 55128 Mainz (DE); MÜLLER, Steffen, 64673 Zwingenberg (DE)
(74) Vertreter: Özer, Alpdeniz
(86) Internationale Anmeldenummer: PCT/EP2016/054616
(87) Internationale Veröffentlichungsnummer: WO 2016/139332

(56) Entgegenhaltungen:
- EP-A1- 2 873 388
- WO-A1-00/76415
- US-A1- 2007 016 176
- US-A1- 2010 167 226
- US-A1- 2014 113 243
- US-A1- 2014 141 389
- US-B1- 6 663 386

## Beschreibung

### Technisches Gebiet

Die Erfindung betrifft einen Dentallaser zur Behandlung von Weichgewebe, der ein Handstück mit einer Applikationsspitze zur Abgabe des Laserlichts umfasst. In der Zahnmedizin wird Weichgewebe, insbesondere Zahnfleisch mit einem Dentallaser geschnitten, der gegenüber einem Dentallaser für die Desinfektion von Zahntaschen oder Wurzelkanälen eine höhere Leistung aufweist.

### Stand der Technik

Aus dem Stand der Technik ist bekannt, Dentallaser zum Schneiden von Weichgewebe einzusetzen, die Laserlicht im Infrarotbereich verwenden. Typische Wellenlängen sind hier 810 ± 15 nm, 940 nm oder 975 ± 15 nm. Die Laserstrahlen werden dabei in einen Lichtwellenleiter der Applikationsspitze eingeleitet und das distale Ende des Lichtwellenleiters wird mit dem Weichgewebe in Kontakt gebracht. Um das Weichgewebe zu schneiden, werden am distalen Ende der Applikationsspitze Leistungen im Bereich von 2-6 W verwendet, die nicht nur im eigentlichen Operationsgebiet, sondern auch im angrenzenden Gewebe zu einer starken Erwärmung des Gewebes führen.

Es ist weiterhin bekannt, Diodenlaser für weitere dentale Anwendungen einzusetzen. So sind aus der WO 2009/003014 A2 eine Vielzahl von Dentallasern bekannt, beispielsweise ein Diodenlaser zur Behandlung von Weichgewebe mit einer großen Koagulationszone für eine bessere Gerinnung, der eine Wellenlänge im Bereich von 500-1350 nm und einer Leistung von 1-100W aufweist. Ein anderer aus der WO 2009/003014 A2 bekannter Dentallaser zur Behandlung von Weichgewebe mit einer geringen Wirkungszone und einem genauen Gewebeschnitt weist Wellenlängen von 300-450 nm oder 1350-3000nm und eine Leistung von 0,1-100 W auf. Noch ein anderer aus der WO 2009/003014 A2 bekannter Diodenlaser mit mehreren Wellenlängen von 410 nm, 577 nm, 975 nm, 1470 nm, 1890 nm und 2940 nm ist offenbart, bei dem diese Wellenlängen verschiedene Maxima der Blut- und Wasserabsorption abdecken, wobei das Wasser als der hauptsächliche Absorber im Weichgewebe des Mundes angesehen wird. Die Laserstrahlen treten über eine Spitze aus dem Handstück aus. Zwar ist von anderen Lasern zur Bearbeitung von Zahnhartsubstanz wie Er:YAG, Er:YSGG bekannt, dass Flüssigkeit zur Kühlung bzw. Prozessunterstützung zugeführt wird. Eine Kühlung der Präparationsstelle mittels Wasser scheidet immer dann aus, wenn die genutzte Wellenlänge sehr stark von Wasser absorbiert wird. Bei den Diodenlasern, deren genutzte Wellenlängen eine hohe Wasserabsorption haben, ist daher keine Kühlung vorgesehen.

Alle bisher bekannten dentalen Diodenlaser werden im sogenannten "Kontaktmode" betrieben, was bedeutet, dass die Applikationsfaser direkt mit dem Gewebe, das zu schneiden ist, in Kontakt, also in Berührung steht. Das Schneiden des Gewebes erfolgt dabei hauptsächlich durch die thermische Wirkung der Applikationsfaser. Die für das Schneiden erforderliche hohe Temperatur an der Applikationsfaser wird erzielt, in dem einerseits ausreichend Laserleistung zur Verfügung gestellt wird und andererseits die Applikationsfaser vor der Behandlung konditioniert wird. Konditioniert werden kann die Faser z.B. dadurch, dass die Oberfläche der Faser z.B. mittels eines Papiers geschwärzt wird. Dazu wird Faser und Papier in direkten Kontakt gebracht und der Laser aktiviert. An der jetzt geschwärzten Oberfläche der Applikationsfaser wird mehr Licht aus der Laserquelle absorbiert und somit die Faser stärker aufgeheizt.

Das Schneiden des Gewebes wird auch durch Absorption der aus der Applikationsfaser austretenden Laserstrahlung im Gewebe unterstützt. Diese Absorption hängt unter anderem von der Wellenlänge des Laserlichts ab. So hat man festgestellt, dass bei einer Wellenlänge von 940 nm eine bessere Absorption der Laserstrahlung im Weichgewebe erfolgt als bei 970 nm.

Das Schneiden mit Kontakt bringt prinzipbedingt immer mit sich, dass ein hoher Wärmeeintrag in das Gewebe erfolgt, der zum Schneiden einerseits notwendig ist, aber andererseits auch zu einer thermischen Schädigung oder zumindest einer großen Belastung des Gewebes selbst und/oder der umliegenden Strukturen, hier sind auch Knochenstrukturen gemeint, führen kann. Gleichwohl sind keine dentalen Diodenlaser bekannt, die berührungslos arbeiten. Das Dokument WO00/76415 offenbart ein Verfahren und eine Vorrichtung zur Auswahl der Wellenlänge für eine Laserbehandlung.

### Darstellung der Erfindung

Ein erfindungsgemäßer Dentallaser weist ein Handstück mit einem Griffbereich und mit einer Behandlungsspitze mit einer an einem distalen Ende angeordneten Austrittsstelle für Laserlicht auf und umfasst weiterhin ein in dem Handstück angeordnetes Lichtleitmittel zur Bereitstellung von aus einer innerhalb oder außerhalb des Handstücks angeordneten Lichtquelle stammendes Laserlicht an der Austrittsstelle. Das Laserlicht weist eine Wellenlänge von 445 ± 20 nm, insbesondere 445 ± 10 nm und weiter insbesondere 445 ± 5 nm, auf und an der Austrittsstelle ist eine optische Leistung in einem Leistungsbereich von mindestens 2 W bis höchstens 5 W, vorteilhafterweise mindestens 3 W und insbesondere 3,5 W bereitgestellt.

Mit optischen Leistungen im Bereich ab 2 W und insbesondere mit 3,5 W ergib sich die Möglichkeit, den Laser berührungslos zu betreiben bei einer guten bis sehr guten Schneideffizienz mit stark reduzierter thermischer Schädigung, da die Absorption von 445 nm direkt im Gewebe besser ist. Die Absorption im Gewebe findet bei 445 nm um Größenordnungen stärker im Hämoglobin statt als in Wasser. Bei anderen Wellenlängen wie 808 nm, 810 nm , 940 nm, 970 nm, 980nm liegen die Absorptionskuren von Wasser und Hämoglobin um Zehnerpotenzen näher zusammen.

Die thermische Schädigung ist insbesondere deswegen geringer, weil der Wärmeeintrag durch die erhitzte Applikationsfaser selbst fehlt und ausschließlich Wärme durch die Absorption der Laserstrahlung im Gewebe zur Gewebeerhitzung beiträgt. Als Obergrenze sind 5W anzusehen, um einen ausreichenden Abstand zu Leistungen mit Schädigungen des Weichgewebes sicherzustellen.

Dadurch, dass beim Schneiden der Wärmeeintrag durch die erhitzte Applikationsfaser selbst fehlt und dass das zu schneidende Gewebe einen sehr hohen Wasseranteil aufweist, kann während des Schneidens Wasser aus dem Gewebe austreten, das nicht wie bei anderen Wellenlängen wie 808 nm, 810 nm, 940 nm, 970 nm, 980nm direkt verdampft. Da Wasser Licht mit einer Wellenlänge von 445nm kaum absorbiert, wird das Wasser durch die Laserstrahlung nicht nennenswert erwärmt oder verdampft. Somit kühlt die austretende Gewebeflüssigkeit selbst zusätzlich die Präparationsstelle.

Durch den fehlenden Kontakt der Faser zur Präparationsstelle und der sehr geringen Absorption des Laserlichts in Wasser kann auch eine externe Wasserkühlung für die Bearbeitungsstelle eingesetzt werden, ohne dass es alleine durch das Therapieinstrument zu Verdampfungen, d.h. zur Reduzierung der Kühlleistung kommt. Bei einer externen Kühlung kann das Kühlmedium wie z.B. Wasser am proximalen Ende der Applikationsfaser an die Applikationsfaser geführt werden und von dort z.B. durch die Schwerkraft an das distale Ende der Applikationsfaser fließen. Es können aber auch vom Handstück aus kleinste Mengen pulsierend in Richtung des distalen Endes der Applikationsfaser mittels Druck gefördert werden und feine Tropfen bilden.

Somit kann das Weichgewebe mit einer Wellenlänge von 445nm und einer optischen Leistung im Bereich ab 2 W und insbesondere mit 3,5 W besonders gut, effizient und schonend geschnitten werden.

Vorteilhafterweise kann die optische Leistung an der Austrittstelle zu einem anderen Leistungsbereich hin veränderbar sein, wobei der andere Leistungsbereich von mindestens 1W bis weniger als 2 W verläuft. Mit geringen Laserleistungen von 1-2 W ist der Dentallaser im Kontaktmode einsetzbar.

Aufgrund der beschriebenen Absorptionsmechanismen ergibt sich ein geeignetes therapeutisches Fenster für die Anwendung eines blauen Diodenlasers mit einer Laserleistung im Bereich von 1-3,5 W und sogar mehr.

Ein weiterer Gegenstand der Erfindung ist ein Dentallaser wie vorstehend beschrieben, wobei jedoch das Laserlicht eine Wellenlänge von 410 ± 10 nm aufweist und an der Austrittsstelle eine optische Leistung in einem Leistungsbereich von mindestens 1 W bis höchstens 2 W bereitgestellt ist.

Bei dieser kürzeren Wellenlänge verbessert sich das Absorptionsverhalten des Laserlichts im Weichgewebe nochmals merklich, sodass eine geringere Leistung ausreichend ist. Dies kann den u.U. erhöhten wirtschaftlichen Aufwand für die Bereitstellung einer Lichtquelle für Laserlicht dieser Wellenlänge rechtfertigen.

Vorteilhafterweise kann die Lichtquelle mindestens eine Laserdiode aufweisen, vorzugsweise mindestens zwei und insbesondere drei.

Dadurch ist es möglich, ein Handstück bereitzustellen, das kompakt ist und eine einfache Handhabung ermöglicht.

Die Laserdioden können sich in einem Gerät befinden, das mittels eines Lichtleiters die Laserstrahlung an das Handstück überträgt. Es ist aber auch möglich, die für die Lichterzeugung erforderliche Laserdiode oder die notwendigen Dioden direkt in das Handstück zu integrieren.

Vorteilhafterweise kann das Lichtleitmittel zur Bereitstellung von Laserlicht an der Austrittsstelle Applikationsfasern zwischen 150- 350 µm Durchmesser aufweisen.

Der Vorteil der Faser zwischen 150-350 µm liegt darin, dass bei den Durchmessern eine relativ hohe Leistung bezogen auf die Austrittsfläche entsteht. Der kleine Durchmesser ermöglicht auch geringe Schnittbreiten, insbesondere dann, wenn die Faser berührungslos über dem zu schneidenden Gewebe geführt wird, da dann die Divergenz der Strahlung zu berücksichtigen ist, die hauptsächlich vom Lichtleitermaterial abhängig ist.

Vorteilhafterweise kann in dem Handstück eine Kühlmittelleitung angeordnet sein und kann eine Auslassöffnung vorhanden sein, aus der Kühlmittel auf die Präparationsstelle gerichtet austritt, wobei die Kühlmittelmenge vorzugsweise zwischen 0,1-10 ml/min beträgt. Durch die Abgabe des Kühlmediums kommt es nicht nur zu einer Kühlung der Präparationsstelle, sondern auch zu einer gezielten Befeuchtung des Gewebes, was für den Heilungsverlauf vorteilhaft ist. Bei zu großer Kühlmittelmenge wird die Laserleistung am Kühlmittel trotz nur geringer Absorption abgeschwächt, mit 0,1 ml/min kann eine Befeuchtung sichergestellt werden. Vorteilhafterweise kann das Kühlmittel desinfizierende Wirkung haben. Damit ist zusätzlich zu einer Kühlung auch eine Desinfektion der Präparationsstelle möglich.

Mit der vorliegenden Erfindung wird ein Dentallaser bereitgestellt, der bei der chirurgischen Behandlung des menschlichen oder tierischen Körpers eingesetzt werden kann, wobei die Nebenwirkungen verringert sind und davon auszugehen ist, dass die Wundheilung schneller erfolgen wird.

### Kurzbeschreibung der Zeichnung

In der Zeichnung ist die Erfindung näher erläutert. Es zeigt:
- Fig. 1: einen schematischen Aufbau eines erfindungsgemäßen Dentallasers mit einer Austrittsstelle des Laserlichts aus einer Applikationsfaser;
- Fig. 2: den Dentallaser aus Fig. 1 mit einer Austrittsstelle des Laserlichts im Freistrahl.

### Ausführungsbeispiel

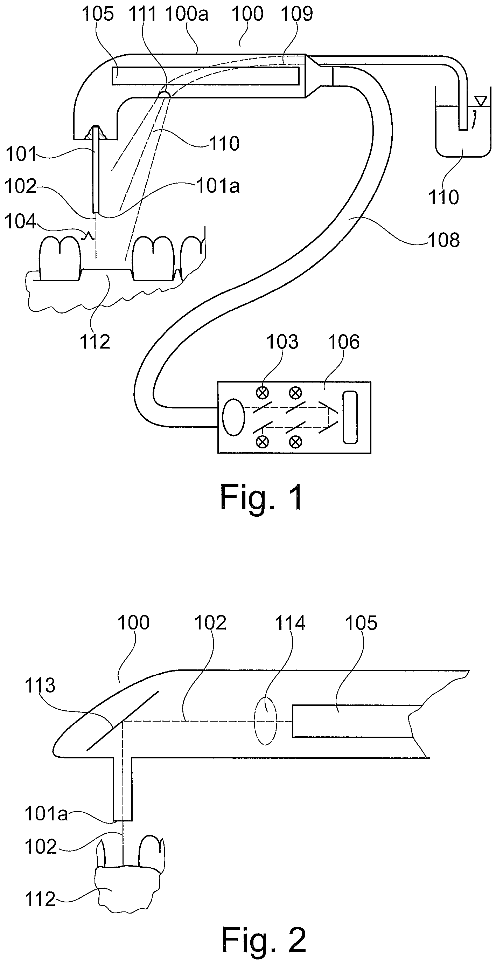

Fig. 1 zeigt einen Dentallaser mit einem Handstück 100 zur Behandlung des menschlichen oder tierischen Körpers mittels Laserstrahlen 102. Die Laserstrahlen 102 werden mittels einer oder mehrerer Laserdioden 103 mit Laserlicht einer Wellenlänge 104 von 445 ± 20 nm, vorzugsweise 445 ± 10 nm besonders vorzugsweise von 445 ± 5 nm in einem Lasermodul 106 erzeugt und über einen Lichtleiter 108 zum Handstück 100 übertragen. Das Handstück 100 weist ein Gehäuse 101 mit einem Griffbereich 100a und eine Behandlungsspitze 101 mit einer an einem distalen Ende angeordneten Austrittsstelle 101a für Laserlicht 102 auf.

In dem Gehäuse ist ein Lichtleitmittel 105 angeordnet zur Bereitstellung von aus einer innerhalb oder außerhalb des Handstücks 100 angeordneten Lichtquelle in Form einer oder mehrerer Laserdioden 103 stammendes Laserlicht an der Austrittsstelle 103.

Gemäß einer bevorzugten Ausführungsform werden drei in einem vom Handstück 100 separaten Lasermodul 106 angeordnete Laserdioden mit einer Wellenlänge von 445 nm ± 20 nm mit jeweils 1,6 W optischer Leistung zu einem Laserstrahl 102 gekoppelt und über einen Lichtleiter 108 in das Handstück 100 geleitet. Damit ergibt sich eine nominelle Leistung von 3 x 1,6 W = 4,8 W. Durch Verluste innerhalb des Lasermoduls 106, hervorgerufen durch die optischen Bauelemente, durch Koppelverluste zwischen dem Lasermodul 106 und der Übertragfaser 108 zum Handstück 100 sowie durch Verluste im Handstück 100 selbst und bei der Ankoppelung der Behandlungsspitzen 101 bleiben am distalen Ende der Behandlungsspitze noch ca. 3,5 W Leistung zur Verfügung.

In der Behandlungsspitze kann eine Applikationsfaser vorhanden sein, an deren distalen Ende das Laserlicht austritt. Es ist aber auch möglich, das Laserlicht im Freistrahl ohne Lichtleiter auf die Behandlungsstelle zu richten. Dies ist in Fig. 2 dargestellt. Ausgehend von dem Lichtleitmittel 105 verläuft ein freier Laserstrahl 102 innerhalb des Handstücks 100 und tritt nach einer Strahlumlenkung mittels eines optischen Bauteils 113 wie einem Spiegel an einer Austrittsstelle 101a aus dem Handstück aus. Nach dem Lichtleitmittel 105 können Mittel 114 vorgesehen sein, beispielsweise optische Mittel wie Linsen, die geeignet sind, die Divergenz der Laserstrahlung zu reduzieren. Damit wird es möglich, den notwenigen Arbeitsabstand der Austrittsstelle 101a zur Präparationsstelle 112 zu erweitern, sodass sich der Abstand, mit dem gut gearbeitet werden kann, über einen vergrößerten Bereich erstreckt.

Die elektrische Leistung des Lasermoduls bei einer nominellen Leistung der Dioden von jeweils 4,8 W beträgt dann 17,2 W, nämlich 3 ^{∗} 4,8 V ^{∗} 1,2 A.

Darüber hinaus ist es möglich die Präparationsstelle mit einer extern zugeführten Kühlflüssigkeit zu kühlen. Wasser ist dazu geeignet, aber auch physiologische Kochsalzlösung wäre dazu geeignet und hätte den Vorteil, dass diese steril leicht verfügbar ist.

Das Handstück 100 kann eine Kühlmittelleitung 109 aufweisen, über welche ein Kühlmittel 110 zu einer Auslassöffnung 111 geführt werden kann, wo das Kühlmittel 110 auf die Präparationsstelle 112 gerichtet austritt.

Die Kühlmenge kann vorzugsweise zwischen 0,1-10 ml/min betragen. Die Kühlung kann vorzugsweise durch Wasser bzw. durch eine physiologische Kochsalzlösung erfolgen.

Mit dem Einsatz der neuen Wellenlänge, 445 nm, blaues Licht, und der erhöhten optischen Laserleistung besteht aufgrund des Absorptionsverhaltens des Weichgewebes die Möglichkeit berührungslos zu schneiden.

Das blaue Licht wird nicht hauptsächlich am Wasser absorbiert sondern am Hämoglobin, das sich ebenfalls im Gewebe befindet. Bei einer Wellenlänge von 445 nm ist der Absorptionskoeffizient für Hämoglobin um das 10⁵-fache höher als der Absorptionskoeffizient für Wasser. Durch dieses Absorptionsverhalten ist es möglich, auch ohne die thermische Übertragung von Energie von der Faser auf das Gewebe zu schneiden. Das Gewebe wird alleine durch die Energie, die auf Grund der Strahlung im Gewebe erzeugt wird, bearbeitet.

Das zum Schneiden verwendete Laserlicht kann auch dadurch bereitgestellt werden, dass die Laserdiode im Handstück direkt sitzt und die Applikationsfaser an die Laserdiode angekoppelt wird. Die Erfindung ist von der Erzeugung und Übertragung des Laserlichts unabhängig.

Bei einer Laserdiode mit einer Leistung von beispielsweise 3,5 W kann diese direkt im Handstück Anwendung finden. Die Übertragungsverluste sind bei einer Platzierung der Laserdiode im Handstück sehr gering, da nur eine Schnittstelle zur Applikationsfaser vorhanden ist.

## Patentansprüche

1. Dentallaser, aufweisend ein Handstück (100) mit einem Griffbereich (100a) und mit einer Behandlungsspitze (101) mit einer an einem distalen Ende angeordneten Austrittsstelle (101a) für Laserlicht (102), weiterhin umfassend ein in dem Handstück (100) angeordnetes Lichtleitmittel (105) zur Bereitstellung von aus einer innerhalb oder außerhalb des Handstücks angeordneten Lichtquelle (103) stammendes Laserlicht (102) an der Austrittsstelle (101a), **dadurch gekennzeichnet, dass** das Laserlicht (102) eine Wellenlänge (104) von 445 ± 20 nm, insbesondere 445 ± 10 nm; und weiter insbesondere 445 ± 5 nm aufweist und dass an der Austrittsstelle (101a) eine optische Leistung in einem Leistungsbereich von mindestens 2 W bis höchstens 5 W, vorteilhafterweise mindestens 3 W und insbesondere 3,5 Watt bereitgestellt ist.

2. Dentallaser nach Anspruch 1, **dadurch gekennzeichnet, dass** die optische Leistung an der Austrittsstelle (101a) zu einem anderen Leistungsbereich hin veränderbar ist, wobei der andere Leistungsbereich von mindestens 1W bis weniger als 2 W verläuft.

3. Dentallaser, aufweisend ein Handstück (100) mit einem Griffbereich (100a) und mit einer Behandlungsspitze (101) mit einer an einem distalen Ende angeordneten Austrittsstelle (101a) für Laserlicht (102), weiterhin umfassend ein in dem Handstück (100) angeordnetes Lichtleitmittel (105) zur Bereitstellung von aus einer innerhalb oder außerhalb des Handstücks angeordneten Lichtquelle (103) stammendes Laserlicht (102) an der Austrittsstelle (101a), **dadurch gekennzeichnet, dass** das Laserlicht (102) eine Wellenlänge (104) von 410 ± 10 nm aufweist und dass an der Austrittsstelle (101a) eine optische Leistung in einem Leistungsbereich von mindestens 1 W bis höchstens 2 W bereitgestellt ist.

4. Dentallaser nach einem der Ansprüche 1 bis 3, **dadurch gekennzeichnet, dass** die die Lichtquelle mindestens eine Laserdiode (103) aufweist, vorzugsweise mindestens zwei und insbesondere drei.

5. Dentallaser nach einem der Ansprüche 1 bis 4, **dadurch gekennzeichnet, dass** die Behandlungsspitze (101) zur Bereitstellung von Laserlicht (102) an der Austrittsstelle (101a) Applikationsfasern zwischen 150-350 µm Durchmesser aufweist.

6. Dentallaser nach einem der Ansprüche 1 bis 5, **dadurch gekennzeichnet, dass** am oder im Handstück (100) eine Kühlmittelleitung (109) angeordnet ist und dass eine Auslassöffnung (111) vorhanden ist, aus der Kühlmittel (110) gerichtet austritt, wobei die Kühlmenge zwischen 0,1-10 ml/min beträgt.

7. Dentallaser nach Anspruch 6, **dadurch gekennzeichnet, dass** das Kühlmittel (110) desinfizierende Wirkung hat.

## Claims

1. Dental laser comprising a handpiece (100) having a grip region (100a) and having a treatment tip (101) with an exit point (101a) for laser light (102) disposed at a distal end, and further comprising a light conduction means (105) disposed in the handpiece (100) for providing laser light (102) at the exit point (101a), which originates from a light source (103) disposed inside or outside the handpiece, **characterized in that** the laser light (102) has a wavelength (104) of 445 ± 20 nm, in particular 445 ± 10 nm, and more particularly 445 ± 5 nm, and that optical power in a power range of at least 2 W to at most 5 W, advantageously at least 3 W and in particular 3.5 Watts, is provided at the exit point (101a) .

2. Dental laser according to Claim 1, **characterized in that** the optical power at the exit point (101a) can be changed to a different power range, wherein the different power range extends from at least 1 W to less than 2 W.

3. Dental laser comprising a handpiece (100) having a grip region (100a) and having a treatment tip (101) with an exit point (101a) for laser light (102) disposed at a distal end, and further comprising a light conduction means (105) disposed in the handpiece (100) for providing laser light (102) at the exit point (101a), which originates from a light source (103) disposed inside or outside the handpiece, **characterized in that** the laser light (102) has a wavelength (104) of 410 ± 10 nm, and that optical power in a power range of at least 1 W to at most 2 W is provided at the exit point (101a).

4. Dental laser according to any one of Claims 1 to 3, **characterized in that** the light source comprises at least one laser diode (103), preferably at least two and in particular three.

5. Dental laser according to any one of Claims 1 to 4, **characterized in that**, at the exit point (101a), the treatment tip (101) comprises application fibers having a diameter between 150 -350 µm for providing laser light (102).

6. Dental laser according to any one of Claims 1 to 5, **characterized in that** a coolant line (109) is disposed on or in the handpiece (100) and that an outlet opening (111) is provided from which coolant (110) exits in a directed manner, wherein the cooling quantity is between 0.1 and 10 ml/min.

7. Dental laser according to Claim 6, **characterized in that** the coolant (110) has a disinfecting effect.

## Revendications

1. Laser dentaire, présentant une pièce à main (100) dotée d'une partie de préhension (100a) et dotée d'une pointe de traitement (101) avec un point de sortie (101a) disposé à une extrémité distale pour de la lumière laser (102), comprenant en outre un moyen de guidage de la lumière (105) disposé dans la pièce à main (100)pour la mise à disposition d'une lumière laser (102) provenant d'une source lumineuse (103) disposée à l'intérieur ou à l'extérieur de la pièce à main au niveau du point de sortie (101a), **caractérisé en ce que** la lumière laser (102) présente une longueur d'onde (104) de 445 ± 20 nm, notamment de 445 ± 10 nm, et plus particulièrement de 445 ± 5 nm et qu'une puissance optique dans un domaine de puissance d'au moins 2 W à au plus 5 W, de manière avantageuse d'au moins 3 W, et notamment 3,5 Watt est mise à disposition au niveau du point de sortie (101a).

2. Laser dentaire selon la revendication 1, **caractérisé en ce que** la puissance optique au niveau du point de sortie (101a) peut être modifiée pour un autre domaine de puissance, où l'autre domaine de puissance s'étend à partir d'au moins 1 W à moins de 2 W.

3. Laser dentaire, présentant une pièce à main (100) dotée d'une partie de préhension (100a) et dotée d'une pointe de traitement (101) avec un point de sortie (101a) disposé à une extrémité distale pour de la lumière laser (102), comprenant en outre un moyen de guidage de la lumière (105) disposé dans la pièce à main (100)pour la mise à disposition d'une lumière laser (102) provenant d'une source lumineuse (103) disposée à l'intérieur ou à l'extérieur de la pièce à main au niveau du point de sortie (101a), **caractérisé en ce que** la lumière laser (102) présente une longueur d'onde (104) de 410 ± 10 nm et qu'au niveau du point de sortie (101a), une puissance optique dans un domaine de puissance d'au moins 1 W à au plus 2 W est mise à disposition.

4. Laser dentaire selon l'une des revendications 1 à 3, **caractérisé en ce que** la source lumineuse présente au moins une diode laser (103), de préférence au moins deux diodes et en particulier trois.

5. Laser dentaire selon l'une des revendications 1 à 4, **caractérisé en ce que** la pointe de traitement (101) présente des fibres d'application d'un diamètre entre 150 et 350 µm pour la mise à disposition de lumière laser (102) au niveau du point de sortie (101a).

6. Laser dentaire selon l'une des revendications 1 à 5, **caractérisé en ce qu'**une conduite de produit réfrigérant (109) est disposée sur ou dans la pièce à main (100), et qu'un orifice de sortie (111) est présent d'où s'échappe le produit réfrigérant (110) de manière orientée, la quantité de réfrigérant étant entre 0,1 et 10 ml/min.

7. Laser dentaire selon la revendication 6, **caractérisé en ce que** le produit réfrigérant (110) possède un effet désinfectant.
